(19)

Europäisches
Patentamt
European
Patent Office
Office européen
des brevets

(11)     **EP 2 087 836 B1**

(12)                 **EUROPEAN PATENT SPECIFICATION**

(45) Date of publication and mention
of the grant of the patent:
**04.04.2012  Bulletin 2012/14**

(51) Int Cl.:
*A61B 5/021* (2006.01)      *A61B 5/029* (2006.01)

(21) Application number: **08101369.0**

(22) Date of filing: **07.02.2008**

(54) **Apparatus and method for determining a physiological parameter**

Vorrichtung und Verfahren zur Bestimmung eines physiologischen Parameters

Appareil et procédé pour la détermination d'un paramètre physiologique

(84) Designated Contracting States:
**DE ES FR GB IT**

(43) Date of publication of application:
**12.08.2009  Bulletin 2009/33**

(73) Proprietor: **Pulsion Medical Systems AG**
**81829 München (DE)**

(72) Inventor: **Dr. Joeken, Stephan**
**Lörrach 79539 (DE)**

(74) Representative: **Ettmayr, Andreas et al**
**Kehl, Ascherl, Liebhoff & Ettmayr**
**Patentanwälte**
**Friedrich-Herschel-Strasse 9**
**81679 München (DE)**

(56) References cited:
**EP-A- 1 884 189        US-A- 5 797 395**
**US-A1- 2005 267 379    US-A1- 2006 167 361**
**US-B1- 6 485 431**

**Description**

The present invention relates to an apparatus for determining at least one physiological parameter of a patient. In particular, the invention relates to an apparatus for determining at least one physiological parameter of a patient, which comprises at least one pressure sensor device adapted to provide readings of a blood pressure of the patient, storage means for storing said readings as at least one pressure curve over time and evaluation means adapted to determine a mean arterial blood pressure from said pressure curve and to determine said at least one physiological parameter using said mean arterial blood pressure.

In addition, the invention relates to a method for determining at least one physiological parameter of a patient, providing readings of a blood pressure of the patient, storage means for storing said readings as at least one pressure curve over time and evaluation means adapted to determine a mean arterial blood pressure from said pressure curve and to determine said at least one physiological parameter using said mean arterial blood pressure.

Furthermore, the invention relates to a physical storage medium having stored thereon a computer program which, when executed on a computer system, causes the computer system to perform a method for determining at least one physiological parameter of a patient.

Devices and methods for determining cardiac function parameters such as cardiac output, ejection fractions or stroke volume are well known in the state of the art. Specifically, cardiac output is obtainable from continuous blood pressure waveforms, which are derived by conventional means via an arterial line measuring the arterial blood pressure. Generally, the cardiac output is equal to the stroke volume multiplied by the heart rate.

For example, EP-A-0 920 278 describes a method for the measurement of cardiac output in a patient, which involves the continuous measurement of the arterial blood pressure waveform, then running an autocorrelation of the waveform data and finally establishing the cardiac output by multiplying the transformed autocorrelation data with a calibration factor using a known accurate calibration method for that patient.

EP-A-0 947 941 describes in vivo determination of the compliance function and the cardiac output of a patient using pulse contour analysis.

However, the current reliable methods for continuously determining cardiac output and/or stroke volume require calibration to a reference value, which usually is determined by an additional discontinuous measurement. In addition, pulse contour analyses require the reliable detection of the dicrotic notch to separate the systolic from the diastolic pulse pressure components. Furthermore, in some circumstances, such as during major surgery, the waveform may not display a dicrotic notch at all. In addition, the above approaches are compromised by disturbances in the pressure measurement due to arrhythmia or reflections from the arterial branches.

Therefore, it is an object of the present invention to provide an apparatus or method, respectively, of the type described above for measuring a physiological parameter such as cardiac output, ejection fractions or stroke volume, which negotiates the necessity for the calibration of the measurement data with a reference value obtained by an additional discontinuous measurement for the patient.

A further object of the present invention is to provide an apparatus or method, respectively, of the type described above, which does away with the requirement to detect the dicrotic notch.

Additionally, it is a particular object of the current invention to minimize the said measurement disturbances in an apparatus or method as described above due to arrhythmia or reflections from the arterial branches.

According to one aspect of the present invention, these objects are achieved by an apparatus for determining at least one physiological parameter of a patient, said apparatus comprising:

- a pressure sensor device adapted to provide readings of an arterial blood pressure of said patient,
- memory means for storing said readings as a pressure curve P(t) over time t,
- evaluation means adapted to determine a mean arterial blood pressure $\langle P \rangle$ from said pressure curve P(t) and to determine said at least one physiological parameter using said mean arterial blood pressure $\langle P \rangle$, wherein said evaluation means are further adapted
- o determine at least one of a spectral density $S(\omega)$ of said pressure curve and a variance $\langle (\delta P)^2 \rangle$ of said arterial blood pressure,
- to determine at least one model parameter representing an effective value of a heart beat using said mean arterial blood pressure $\langle P \rangle$ and at least one of said spectral density $S(\omega)$ and said variance $\langle (\delta P)^2 \rangle$, said effective value being selected from an effective amplitude $A_{eff}$ of said heart beat, an effective duration $d_{eff}$ of said heart beat and an effective area $F_{eff}$ under said heart beat, and
- to determine said at least one physiological parameter using at least one said model parameter.

Preferably, said at least one physiological parameter includes at least one of stroke volume SV, cardiac output CO and ejection fraction EF.

It is further preferred that said evaluation means are adapted to determine said stroke volume SV as a product of a first model parameter representing said effective amplitude $A_{eff}$ and a second model parameter representing said effective duration $d_{eff}$.

In addition, it is preferred that said evaluation means are adapted to determine said ejection fraction EF as a product of a model parameter representing said effective duration $d_{eff}$ and a heart rate HR of said patient.

In particular preferred is that said evaluation means are adapted to determine said cardiac output CO as a product of a first model parameter representing said effective amplitude $A_{eff}$, a second model parameter representing said effective duration $d_{eff}$ and an approximation of a heart rate of said patient.

Preferably, said approximation of said heart rate is selected from an actual measured heart rate HR and an approximate function $\lambda_{eff}$, said approximate function $\lambda_{eff}$ including a quotient with a dividend comprising the square of the mean arterial pressure <P> and a divisor comprising the spectral density $S(\omega)$ at $\omega=0$.

Further preferably, said evaluation means are adapted to use a correction parameter $\alpha$ in determining said model parameter, said correction parameter $\alpha$ assuming values greater than or equal to 1, said values being the higher, the less the patient's heart frequency deviates from a rhythmic condition.

In addition, it is preferred that said evaluation means are adapted to use a monotonous correction function $\sigma$ depending on said correction parameter $\alpha$ and assuming values from 0 to 1, wherein said correction function $\sigma$ assumes the value of 0 if said correction parameter $\alpha$ equals 1 and said correction function $\sigma$ assumes the value of 1 for said correction parameter $\alpha$ tending to infinity.

Preferably, said effective amplitude $A_{eff}$ is provided as a quotient with a dividend comprising the sum of said variance $<(\delta P)^2>$ and the product of said correction function $\sigma$ and the square of the mean arterial pressure <P> and a divisor comprising the mean arterial pressure <P>.

Furthermore preferably, said effective amplitude $d_{eff}$ is provided as a quotient with a dividend comprising the spectral density $S(\omega)$ at $\omega=0$ and a divisor comprising the sum of said variance $<(\delta P)^2>$ and the product of said correction function $\sigma$ and the square of the mean arterial pressure <P>.

Also preferably, said apparatus provides Fourier Transformation means for determining said spectral density $S(\omega)$ as the Fourier Transformation of the autocorrelation of said pressure curve.

Preferably, the spectral density $S(\omega)$ at $\omega=0$ is provided as said variance $<(\delta P)^2>$ multiplied by a constant factor.

In a preferred embodiment, said evaluation unit is further adapted to determine a comparative value of at least one of said physiological parameters from said pressure curve using pulse contour algorithms.

In an additional preferable embodiment said apparatus further comprises

- means for administering a travelling deviation of an intrinsic physical quantity (such as temperature, an optical/ spectral property or an indicator concentration) to the blood stream of said patient at a first location of the blood circulation of said patient, and
- sensor means for measuring said physical quantity at a second location of the blood circulation of said patient over the course of time,

wherein, said memory means are adapted to record said physical quantity measured over the course of time at said second location as a dilution curve, and said evaluation unit is adapted to determine a comparative value of at least one of said physiological parameters from said dilution curve using dilution algorithms.

Preferably, said evaluation unit is adapted to use said comparative value for calibration.

Preferably, said calibration includes determining, using said comparative value, a correction parameter $\alpha$ used in determining said model parameter, said correction parameter $\alpha$ assuming values greater than or equal to 1, said values being the higher the less the patient's heart frequency deviates from a rhythmic condition.

In a preferred embodiment, said evaluation unit is adapted to reject and re-calculate said physiological parameter, if the difference between the determined physiological parameter and the respective comparative value exceeds a threshold value.

Further to these embodiments, the above objects are achieved under an additional aspect of the invention by a method for determining at least one physiological parameter of a patient, said method comprising the steps of:

- importing readings of an arterial blood pressure of said patient,
- storing said readings as a pressure curve P(t) over time t,
- determining a mean arterial blood pressure <P> from said pressure curve P(t) and determining at least one physiological parameter using said mean arterial blood pressure <P>,

wherein the method further includes

- determining at least one of a spectral density $S(\omega)$ of said pressure curve and a variance $<(\delta P)^2>$ of said arterial blood pressure, determining at least one model parameter representing an effective value of a heart beat using said

3

mean arterial blood pressure $<P>$ and at least one of said spectral density $S(\omega)$ and said variance $<(\delta P)^2>$, said effective value being selected from an effective amplitude $A_{eff}$ of said heart beat, an effective duration $d_{eff}$ of said heart beat and an effective area $F_{eff}$ under said heart beat, and

- determining said at least one physiological parameter using at least one said model parameter.

Preferably, in above method said at least one physiological parameter includes at least one of stroke volume SV, cardiac output CO and ejection fraction EF.

Furthermore preferably in above method, said stroke volume SV is determined as a product of a first model parameter representing said effective amplitude $A_{eff}$ and a second model parameter representing said effective duration $d_{eff}$.

It is further preferred that in above method said ejection fraction EF is determined as a product of a model parameter representing said effective duration $d_{eff}$ and a heart rate HR of said patient.

Further preferably, in above method said cardiac output CO is determined as a product of a first model parameter representing said effective amplitude $A_{eff}$, a second model parameter representing said effective duration $d_{eff}$ and an approximation of a heart rate of said patient.

Also preferably in above method, said approximation of said heart rate is selected from an actual measured heart rate HR and an approximate function $\lambda_{eff}$. said approximate function $\lambda_{eff}$ including a quotient with a dividend comprising the square of the mean arterial pressure $<P>$ and a divisor comprising the spectral density $S(\omega)$ at $\omega=0$.

In addition, in above method, it is preferred that a correction parameter $\alpha$ is used in determining said model parameter, said correction parameter $\alpha$ assuming values greater than or equal to 1, said values being the higher the less the patient's heart frequency deviates from a rhythmic condition.

Preferably, in above method, a monotonous correction function $\sigma$ is used depending on said correction parameter $\alpha$ and assuming values from 0 to 1, wherein said correction function $\sigma$ assumes the value of 0 if said correction parameter $\alpha$ equals 1 and said correction function $\sigma$ assumes the value of 1 for said correction parameter $\alpha$ tending to infinity.

In a preferred embodiment of above method, said effective amplitude $A_{eff}$ is provided as a quotient, the dividend of said quotient comprising the sum of said variance $<(\delta P)^2>$ and the product of said correction function $\sigma$ and the square of the mean arterial pressure $<P>$ and the divisor of said quotient comprising the mean arterial pressure $<P>$.

Preferably, in above method, said effective amplitude $d_{eff}$ is provided as a quotient with a dividend comprising the spectral density $S(\omega)$ at $\omega=0$ and a divisor comprising the sum of said variance $<(\delta P)^2>$ and the product of said correction function $\sigma$ and the square of the mean arterial pressure $<P>$.

It is further preferred that in above method, said spectral density $S(\omega)$ is determined as the Fourier Transformation of the autocorrelation of said pressure curve.

Preferably, in above method, the spectral density $S(\omega)$ at $\omega=0$ is provided as said variance $<(\delta P)^2>$ multiplied by a constant factor.

Preferably, in above method, a comparative value of at least one of said physiological parameters is determined from said pressure curve using pulse contour algorithms.

In a preferred embodiment, above method further comprises

- inputting information about a traveling deviation of an intrinsic physical quantity (such as temperature, an optical/spectral property or an indicator concentration) administered to the blood stream of said patient at a first location of the blood circulation of said patient, and
- reading in measurement readings of said physical quantity at a second location of the blood circulation of said patient over the course of time,

wherein, said measurement readings of said physical quantity at said second location of the blood circulation of said patient are recorded as a dilution curve, and said comparative value of at least one of said physiological parameters is determined from said dilution curve using dilution algorithms.

Preferably, in above method, said comparative value is used for calibration.

In addition in above method, it is preferred that said calibration includes determining, using said comparative value, a correction parameter $\alpha$ used in determining said model parameter, said correction parameter $\alpha$ assuming values greater than or equal to 1, said values being the higher the less the patient's heart frequency deviates from a rhythmic condition.

Preferably, in above method, said physiological parameter is rejected and re-calculated, if the difference between the determined physiological parameter and the respective comparative value exceeds a threshold value.

An additional embodiment of the present invention comprises a physical storage medium having stored thereon a computer program which, when executed on a computer system, causes the computer system to perform a method as described above.

Generally, any of the embodiments described or options mentioned herein may be particularly advantageous depending on the actual conditions of application. Further, features of one embodiment may be combined with features of another embodiment as well as features known per se from the prior art as far as technically possible and unless indicated otherwise.

In the following, the invention is described in more detail. The accompanying drawings, which are schematic flow diagrams and graphs, serve for a better understanding of the features of the current invention.

Therein:

Fig. 1    is a flow diagram illustrating a preferred embodiment of the inventive method for determining the cardiac output CO. Optional features are presented in dashed lines. The quantity $\alpha$ may depend on the CVP (central venous pressure) and/or the heart rate variability,

Fig. 2    is an exemplary plot of the functional relationship $\sigma(\alpha)$, i.e. the behaviour of $\sigma$ for typical values of $\alpha$, according to one embodiment of the present invention

Fig. 3    shows a correlation plot between the CO values determined according to an embodiment of the present invention by eq. (24) and the reference values $CO_{ref}$ obtained by the transpulmonary cardiac output measurements.

Fig. 4    illustrates the general setup of an apparatus according to an embodiment of the present invention

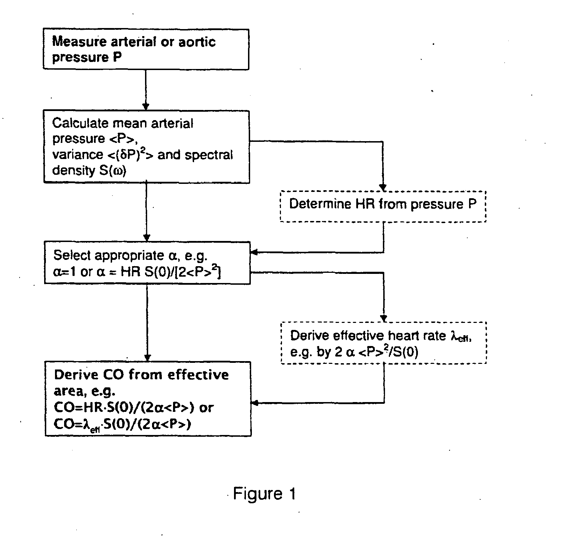

Figure 1 illustrates (indicated by regular lines) the most important steps of one preferred embodiment of the present invention. Dashed lines indicate optional steps for modifying the embodiment to yield another preferred embodiment. In order to improve the illustration of the preferred embodiments and to ease the understanding thereof, the following description also attempts to describe in more detail the underlying calculatory principles.

While the main focus of the described embodiments is on cardiac output determination, other physiological parameters may also be established with the apparatus and methods according to the invention, in particular, such parameters characterising cardiac function, such as ejection fractions or stroke volume.

As is shown in figure 1, the current invention makes use of measurement readings representing the arterial or aortic pressure $P$, *the readings being provided either as raw data (such* as *voltage or electric current) from a suitable sensor or already in a pre-processed state.* Performing the actual measurements may be achieved in many ways, such as employing a suitable arterial catheter assembly with a pressure transducer or applying non-invasive blood pressure measurements, and is well known from the prior art and not part of the inventive method itself. The measured pressure depends on time $t$ and results from the superposition of several heart beats $b_k(t - t_k)$, beginning at time $t_k$. Due to reflections in the arterial tree, several heart beats may contribute to the resulting pressure, which can be described by the following function:

$$P(t) = \sum_k b_k(t - t_k) \tag{1}$$

In general, all beats may differ from each other, however, for a limited period of time, an average beat b(t) exists so that the function of equation (1) can be described adequately by

$$P(t) = \sum_k b_k(t - t_k) \tag{2}$$

$$\approx \sum_k b(t - t_k) \tag{3}$$

As in healthy living beings the heart rate HR ususally does not vary strongly within a limited time frame (e.g. 10 s to 200 s), successive beats are almost equidistant and the occurrence of beats is determined by $t_k = k/HR$.

During strong arrhythmia, however, heart beats occur very irregularly. The worst case scenario is the random occurrence of independent beats, which can be described by a Poisson-Process, i.e. the intervals between successive heart beats. are distributed at random according to an exponential-distribution $q$ such that:

$$q(t_k - t_{k-1}) = HR * exp(-HR * (t_k - t_{k-1})).$$

In this case, the superposition of equation (3) is a Campbell-Process, as is described in: [N. R. Campbell, "The study of discontinuous phenomena." Proc. Camp. Philos. Soc. Math. Phys. Sci. 15:117-136, 1909.]

According to the present embodiment, the cardiac function can be assessed in both conditions, i.e. for regular and irregular heart activity, by means of the mean, variance and spectral density of the measured pressure function $P(t)$,

The mean arterial pressure $<P>$ is derived from the integral of the measured pressure $P(t)$ over an appropriate time span T, i.e.

$$MAP = <P> = \frac{1}{T} \int_0^T P(t)dt \qquad (4)$$

The Variance of the pressure is calculated from $\delta P(t) = P(t) - <P>$ so that:

$$<(\delta P(t))^2> = \frac{1}{T} \int_0^T (P(t) - <P>)^2 dt \qquad (5)$$

The Spectral density is the Fourier Transformation of the autocorrelation of $P(t)$ and hence is determined by:

$$S(\omega) = 4 \int_0^\infty \cos(\omega\tau) <\delta P(t) * \delta P(t - \tau)> d\tau \qquad (6)$$

In the case of arrhythmia, the arrhythmic conditions can be described by a Campbell-Process application of equations (4) to (6), which results in the following functions:

$$<P> = HR * \int_0^\infty b(t)dt \qquad (7)$$

$$<(\delta P)^2> = HR * \int_0^\infty b^2(t)dt \qquad (8)$$

$$S(\omega) = 2 * HR * |\tilde{b}(\omega)|^2 \qquad (9)$$

with the Fourier Transformation $b(\omega)$ of the average beat $b(t)$:

$$\tilde{b}(\omega) \; = \; \int_{0}^{\infty} b(t) * e^{-i\omega t} dt \qquad\qquad (10)$$

For rhythmic conditions with periodic heart beats, the application of equations (4) to (6) results to:

$$<P> \; = \; HR * \int_{0}^{\infty} b(t) dt \qquad\qquad (11)$$

$$<(\delta P)^2> \; = \; HR * \int_{0}^{\infty} b^2(t) dt \; - \; <P>^2 \qquad\qquad (12)$$

$$S(\omega) \; = \; 2\alpha * HR * \left| \tilde{b}(\omega) \right|^2, \qquad \text{with} \; \alpha > 1 \qquad (13)$$

The parameter $\alpha$ depends on the heart rate variability on a time scale much larger than T. For practical implementations, $\alpha$ can be determined through imperical studies or simply estimated taking into account the principles described herein.

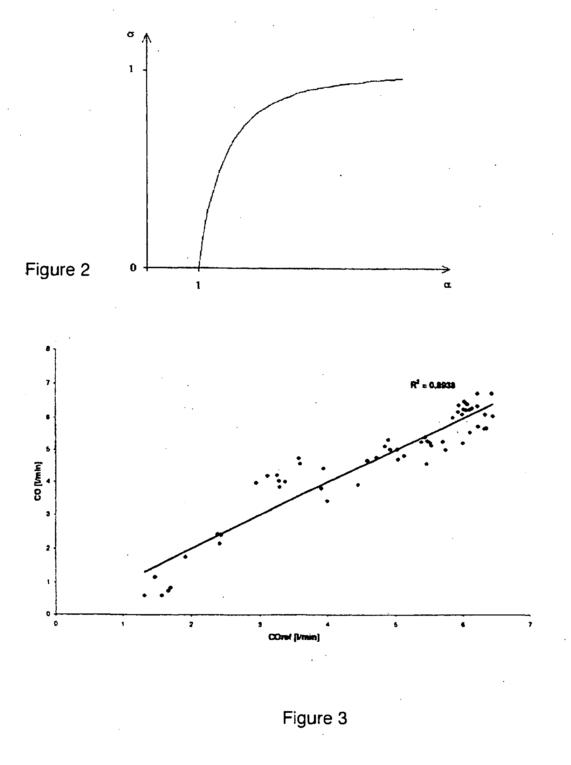

Merging the different conditions can be based on the following considerations. Equations (7) to (9) and (11) to (13) have the same structure. In particular, $\alpha$ in eq. (13) decreases with a decreasing accuracy in the periodicity of the beats *b(t)*. Finally, for randomly occurring beats, $\alpha$ tends to the lower limit $\alpha = 1$ and the equations (13) and (11) become identical. Equations (12) and (8) can also be merged. Therefore, a function $\sigma(\alpha)$ is introduced, which tends to 0 as $\alpha$ tends to 1, and $\sigma(\alpha)$ tends to 1 for $\alpha >> 1$. This relationship is described in an exemplary manner in figure 2.

In consequence, the rhythmic, arrhythmic and intermediate activity of the heart is described by:

$$<P> \; = \; HR * \int_{0}^{\infty} b(t) dt \qquad\qquad (14)$$

$$<(\delta P)^2> \; = \; HR * \int_{0}^{\infty} b^2(t) dt \; - \; \sigma(\alpha) <P>^2 \qquad\qquad (15)$$

$$S(\omega) \; = \; 2\alpha * HR * \left| \tilde{b}(\omega) \right|^2 \qquad\qquad (16)$$

wherein $\alpha$ and $\sigma$ are limited as follows:

|  | $\alpha$ | $\sigma$ (alpha) |
|---|---|---|
| arrhythmic | 1 | 0 |
| intermediate | $\geq 1$ | > 0 and < 1 |
| rhythmic | >1 | 1 |

Furthermore, the acquired data may be interpreted so as to afford a characterisation of the average beat in terms of its amplitude A and duration *d*.

$$A := \frac{\int b^2(t)dt}{\int b(t)dt} \qquad (17)$$

$$d := \frac{\int b(t)dt \,^2}{\int b^2(t)dt} \qquad (18)$$

Thus, if a square wave is assumed,

$$b(t) = A, \qquad for\ 0 \le t \le d$$
$$b(t) = 0, \qquad otherwise \qquad (19)$$

Consequently, introducing the equations (14) to (16) yields:

$$A_{eff} = \frac{<(\delta P)^2> + \sigma <P>^2}{<P>} \qquad (20)$$

$$d_{eff} = \frac{S(0)}{2\alpha^*(<(\delta P)^2> + \sigma <P>^2)} \qquad (21)$$

Therein, the index "*eff*" was introduced to point out that $A_{eff}$ and $d_{eff}$ are effective values reconstructed from the measured pressure signal *P(t)*. It is emphasized that this reconstruction is possible in all conditions, even if no heart beats are detectable in the pressure curve *P(t)*. Moreover, the product of the area $A_{eff}$ and duration $d_{eff}$ results in the area under the effective beat $F_{eff}$, which is

$$F_{eff} = A_{eff} * d_{eff}$$

$$= \frac{S(0)}{2\alpha <P>} \qquad (22)$$

even if the beats do not represent a square wave.
In addition, the ratio

$$\lambda_{eff} \quad = \quad \frac{2\alpha <P>^2}{S(0)} \tag{23}$$

gives an approximation of the heart rate in all circumstances.

According to the present embodiments, the obtained parameters are used to characterize a patient's cardiac function. In particular, the quantities $A_{eff}$, $d_{eff}$, $\lambda_{eff}$ and combinations thereof are useful to characterize stroke volume SV, cardiac output CO, ejection fraction EF and others.

Specificalfy, the Cardiac output CO may be derived according to

$$CO \quad = \quad HR \quad * \quad A_{eff} \quad * \quad d_{eff}$$

$$= \quad HR \quad * \quad \frac{S(0)}{2\alpha <P>} \tag{24}$$

In order to test the values obtained with the inventive method, a comparison with a known standard reference method was undertaken. For this purpose the cardiac output was measured according to the present invention and also with the established thermodilution technique according to EP-A-0 637 932.

The results of this comparison are displayed in figure 3. Therein, the abscissa represents cardiac output in liters per minute as determined by the reference method and the ordinate represents output in liters per minute as determined by the inventive method. Accordingly, the results achieved for the cardiac output in both measurements are in good agreement.

Furthermore, the other cardiac functions stroke volume and ejection fraction are also readily available from the parameters determined according to the inventive method.

The stroke volume SV is afforded by dividing the cardiac output by the heart rate HR; thus: $SV = A_{eff} * d_{eff}$.

Further, the quantity $d_{eff} * HR$ corresponds to the ejection fraction EF. Depending on the location of the pressure measurement (pulmonary artery or systemic artery) the readings of which are used, this might by either the left or the right ventricular ejection fraction.

Further embodiments of the invention will be described below.

An optional simplification to the inventive method may be made, if the spectral density is required for frequencies $f$ close to or at $\omega = 2\pi f = 0$. Then according to eq. (6), the variance of the pressure P can be used to approximate

$$S(0) \quad \approx \quad 2 \quad * \quad (<P^2> - <P>^2) \tag{25}$$

$$= \quad 2 \quad * \quad <(\delta P)^2> \tag{26}$$

This simplification can also be used in the other relations, particularly, in eq. (24).

In addition, an arrhythmia adjustment may be made since the coefficient $1/\alpha$ may depend on the heart rate variability.

Furthermore, the central venous pressure CVP may influence the coefficient $1/\alpha$. Therefore, in order to establish $\alpha$, CVP can be taken into account either by reading in measurement data directly as provided by a CVP measurement source or by user input. Generally, measuring CVP per se is well known from the prior art and not part of the inventive method.

The effect of a varying heart rate on the current invention can be detected by determining the number of heart beats within a pressure sample and the effective heart rate $\lambda_{eff}$ determined by equation (23). Particularly, the ratio of HR and $\lambda_{eff}$ leads to

$$\alpha = \frac{HR * S(0)}{2 <P>^2} \tag{27}$$

Therefore, an adjustment of a patient monitoring system implementing the present invention to the current heart rate variability is possible.

The Spectral density S(0) for $\omega = 2\pi f = 0$ can also be determined differently, e.g.

a) by means of the spectral density for frequencies tending to zero, i.e. $S(0) = \lim_{\omega \to 0} S(\omega) = \lim_{t \to 0} S(2\pi f)$, or

b) by fitting an appropriate function (e.g. $S(\omega) = S(0) / [1 + (\omega\tau)^2]^{n+1}$) to the measured spectrum. Therein, local maxima corresponding to the heart rate should preferably be neglected.

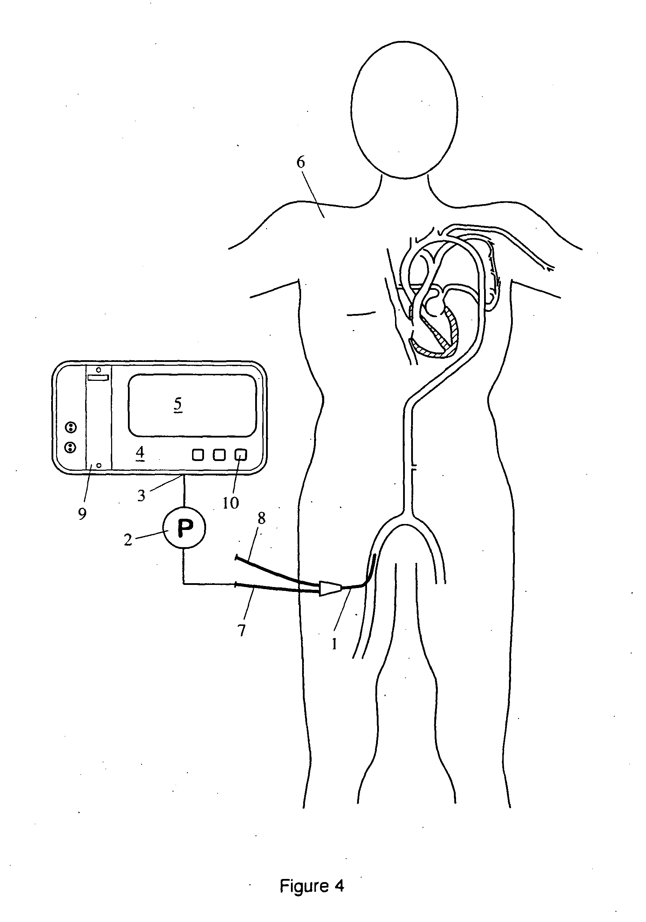

Figure 4 shows the general setup of an apparatus according to an embodiment of the present invention. An arterial catheter 1 is equipped with a pressure sensor for measuring arterial blood pressure. The pressure sensor of the catheter 1 is connected, via a pressure transducer 2, to an input channel 3 of a patient monitoring apparatus 4. Beside a proximal port 7 used to acquire the pressure signal, the catheter 1 may comprise one or more other proximal ports 8 to perform additional functions, such as blood temperature measurements or the like. The patient monitoring apparatus 4 contains appropriate storage means for storing the readings of blood pressure over time and serving as a processing storage.

The patient monitoring also comprises a computing facility 9, which may include a digital signal processing instance 9, which is programmed to perform calculations in accordance with the equations described above, a display 5 to visualize the determined parameters (as numeric values, graphically or both) and control elements 10 to operate said apparatus. In addition, the determined parameters may be stored at a recording medium and/or printed. For this purpose, the patient monitoring apparatus 4 may comprise various interface ports for connecting peripheral equipment.

A particularly preferred embodiment requires a single arterial pressure sensor only. Though the sensor is shown to be invasive using said catheter 1, a non-invasive pressure sensor may be implemented instead. Instead of (or in addition to) an arterial catheter 1 as shown, a pulmonary artery catheter may also be used, in particular, if right ventricular ejection fraction is to be determined.

Various implementations of the invasive pressure sensor assembly can be particularly advantageous. Pressure can either be transmitted hydraulically to a proximal catheter port 7 and measured by an external sensor or may be measured directly on-site using a sensor installed at or near the catheter tip. Capacitative sensors, piezo sonsors or optical pressure sensors (e.g. based on Fabry-Perot interferometry) may be used.

With reference to the procedure described in figure 1, a first pressure signal P is measured by said apparatus depicted in figure 4, e.g. from arteria femoralis, arteria radialis, arteria brachialis or arteria pulmonalis or any other appropriate arterial vessel downstream of the left or right ventricle, respectively.

By means of equation (6), the computing facility 9 calculates the mean arterial pressure, the variance and the spectral density for the pressure measured within an appropriate time frame (e.g. 10 s to 200 s). For calculating the spectral density, designated fast fourier transformation means may be employed.

Furthermore, for $\omega = 0$ and $\alpha = 1$ the computing facility 9 employs equation (22) to calculate the effective area $F_{eff}$. The relationship between the effective area $F_{eff}$ and the stroke volume SV is given by $1/\alpha$, wherein the latter can be determined empirically or alternatively, with respect to reference SV measurements (e.g. transpulmonary thermodilution) from the treated patient. In order to perform such reference SV measurements, the patient monitoring apparatus 4 may be equipped accordingly, e.g. by providing additional input channels for thermodilution measurement readings and by implementing suitable thermodilution algorithms in the computing facility 9, as known per se from the prior art.

The knowledge of SV permits the determination of CO according to *CO = HR * SV,* wherein the heart rate HR can also, optionally, be determined from the pressure and the spectral density or by other methods.

## Claims

1. Apparatus for determining at least one physiological parameter of a patient, said apparatus comprising:

- a pressure sensor device adapted to provide readings of an arterial blood pressure of said patient,
- memory means for storing said readings as a pressure curve P(t) over time t,

- evaluation means adapted to determine a mean arterial blood pressure <P> from said pressure curve P(t) and to determine said at least one physiological parameter using said mean arterial blood pressure <P>, **characterized in that**

said evaluation means are further adapted

- to determine at least one of a spectral density $S(\omega)$ of said pressure curve and a variance $<(\delta P)^2>$ of said arterial blood pressure,
- to determine at least one model parameter representing an effective value of a heart beat using said mean arterial blood pressure <P> and at least one of said spectral density $S(\omega)$ and said variance $<(\delta P)^2>$, said effective value being selected from an effective amplitude $A_{eff}$ of said heart beat, an effective duration $d_{eff}$ of said heart beat and an effective area $F_{eff}$ under said heart beat, and
- to determine said at least one physiological parameter using at least one said model parameter.

2. Apparatus according to claim 1, wherein said at least one physiological parameter includes at least one of stroke volume SV, cardiac output CO and ejection fraction EF.

3. Apparatus according to claim 2, wherein said evaluation means are adapted to determine said stroke volume SV as a product of a first model parameter representing said effective amplitude $A_{eff}$ and a second model parameter representing said effective duration $d_{eff}$.

4. Apparatus according to claim 2 or claim 3, wherein said evaluation means are adapted to determine said ejection fraction EF as a product of a model parameter representing said effective duration $d_{eff}$ and a heart rate HR of said patient.

5. Apparatus according to one of claims 2 to 4, wherein said evaluation means are adapted to determine said cardiac output CO as a product of a first model parameter representing said effective amplitude $A_{eff}$, a second model parameter representing said effective duration $d_{eff}$ and an approximation of a heart rate of said patient.

6. Apparatus according to claim 5, wherein said approximation of said heart rate is selected from an actual measured heart rate HR and an approximate function $\lambda_{eff}$, said approximate function $\lambda_{eff}$ including a quotient with a dividend comprising the square of the mean arterial pressure <P> and a divisor comprising the spectral density $S(\omega)$ at $\omega=0$.

7. Apparatus according to any of the preceding claims, wherein said evaluation means are adapted to use a correction parameter $\alpha$ in determining said model parameter, said correction parameter $\alpha$ assuming values greater than or equal to 1, said values being the higher, the less the patient's heart frequency deviates from a rhythmic condition.

8. Apparatus according to claim 7, wherein said evaluation means are adapted to use a monotonous correction function $\sigma$ depending on said correction parameter $\alpha$ and assuming values from 0 to 1, wherein said correction function $\sigma$ assumes the value of 0 if said correction parameter $\alpha$ equals 1 and said correction function $\sigma$ assumes the value of 1 for said correction parameter $\alpha$ tending to infinity.

9. Apparatus according to claim 8, wherein said effective amplitude $A_{eff}$ is provided as a quotient with a dividend comprising the sum of said variance $<(\delta P)^2>$ and the product of said correction function $\sigma$ and the square of the mean arterial pressure <P> and a divisor comprising the mean arterial pressure <P>.

10. Apparatus according to claim 8 or claim 9, wherein said effective amplitude $d_{eff}$ is provided as a quotient with a dividend comprising the spectral density $S(\omega)$ at $\omega=0$ and a divisor comprising the sum of said variance $<(\delta P)^2>$ and the product of said correction function $\sigma$ and the square of the mean arterial pressure <P>.

11. Apparatus according to any of the preceding claims comprising Fourier Transformation means for determining said spectral density $S(\omega)$ as the Fourier Transformation of the autocorrelation of said pressure curve.

12. Apparatus according to any of the preceding claims, wherein the spectral density $S(\omega)$ at $\omega=0$ is provided as said variance $<(\delta P)^2>$ multiplied by a constant factor.

13. Apparatus according to any of the preceding claims, wherein said evaluation unit is further adapted to determine a comparative value of at least one of said physiological parameters from said pressure curve using pulse contour algorithms.

14. Apparatus according to any of the preceding claims, further comprising

    - means for administering a traveling deviation of an intrinsic physical quantity to the blood stream of said patient at a first location of the blood circulation of said patient, and
    - sensor means for measuring said physical quantity at a second location of the blood circulation of said patient over the course of time,

wherein said memory means are adapted to record said physical quantity measured over the course of time at said second location as a dilution curve, and said evaluation unit is adapted to determine a comparative value of at least one of said physiological parameters from said dilution curve using dilution algorithms.

15. Apparatus according to claim 13 or claim 14 wherein said evaluation unit is adapted to use said comparative value for calibration.

16. Apparatus according to claim 15, wherein calibration includes determining, using said comparative value, correction parameter $\alpha$ used in determining said model parameter, said correction parameter $\alpha$ assuming values greater than or equal to 1, said values being the higher the less the patient's heart frequency deviates from a rhythmic condition.

17. Apparatus according to any of claims 13 to 16, wherein said evaluation unit is adapted to reject and re-calculate said physiological parameter, if the difference between the determined physiological parameter and the respective comparative value exceeds a threshold value.

18. Method for determining at least one physiological parameter of a patient, said method comprising the steps of:

    - importing readings of an arterial blood pressure of said patient,
    - storing said readings as a pressure curve P(t) over time t,
    - determining a mean arterial blood pressure <P> from said pressure curve P(t) and determining at least one physiological parameter using said mean arterial blood pressure <P>

**characterized in that**
said method further includes

    - determining at least one of a spectral density $S(\omega)$ of said pressure curve and a variance $<(\delta P)^2>$ of said arterial blood pressure,
    - determining at least one model parameter representing an effective value of a heart beat using said mean arterial blood pressure <P> and at least one of said spectral density $S(\omega)$ and said variance $<(\delta P)^2>$, said effective value being selected from an effective amplitude $A_{eff}$ of said heart beat, an effective duration $d_{eff}$ of said heart beat and an effective area $F_{eff}$ under said heart beat, and
    - determining said at least one physiological parameter using at least one said model parameter.

19. Method according to claim 18, wherein said at least one physiological parameter includes at least one of stroke volume SV, cardiac output CO and ejection fraction EF.

20. Method according to claim 19, wherein said stroke volume SV is determined as a product of a first model parameter representing said effective amplitude $A_{eff}$ and a second model parameter representing said effective duration $d_{eff}$.

21. Method according to claim 19 or 20, wherein said ejection fraction EF is determined as a product of a model parameter representing said effective duration $d_{eff}$ and a heart rate HR of said patient.

22. Method according to one of claims 19 to 21, wherein said cardiac output CO is determined as a product of a first model parameter representing said effective amplitude $A_{eff}$, a second model parameter representing said effective duration $d_{eff}$ and an approximation of a heart rate of said patient.

23. Method according to claim 22, wherein said approximation of said heart rate is selected from an actual measured heart rate HR and an approximate function $\lambda_{eff}$, said approximate function $\lambda_{eff}$ including a quotient with a dividend comprising the square of the mean arterial pressure <P> and a divisor comprising the spectral density $S(\omega)$ at $\omega=0$.

24. Method according to any of claims 18 to 23, wherein a correction parameter $\alpha$ is used in determining said model parameter, said correction parameter $\alpha$ assuming values greater than or equal to 1, said values being the higher the less the patient's heart frequency deviates from a rhythmic condition.

25. Method according to claim 24, wherein a monotonous correction function $\sigma$ is used depending on said correction parameter $\alpha$ and assuming values from 0 to 1, wherein said correction function $\sigma$ assumes the value of 0 if said correction parameter $\alpha$ equals 1 and said correction function $\sigma$ assumes the value of 1 for said correction parameter $\alpha$ tending to infinity.

26. Method according to claim 25, wherein said effective amplitude $A_{eff}$ is provided as a quotient, the dividend of said quotient comprising the sum of said variance $<(\delta P)^2>$ and the product of said correction function $\sigma$ and the square of the mean arterial pressure $<P>$ and the divisor of said quotient comprising the mean arterial pressure $<P>$.

27. Method according to claim 25 or claim 26, wherein said effective amplitude $d_{eff}$ is provided as a quotient with a dividend comprising the spectral density $S(\omega)$ at $\omega=0$ and a divisor comprising the sum of said variance $<(\delta P)^2>$ and the product of said correction function $\sigma$ and the square of the mean arterial pressure $<P>$.

28. Method according to any of claims 18 to 27, wherein said spectral density $S(\omega)$ is determined as the Fourier Transformation of the autocorrelation of said pressure curve.

29. Method according to any of claims 18 to 28, wherein the spectral density $S(\omega)$ at $\omega=0$ is provided as said variance $<(\delta P)^2>$ multiplied by a constant factor.

30. Method according to any of claims 18 to 29, wherein a comparative value of at least one of said physiological parameters is determined from said pressure curve using pulse contour algorithms.

31. Method according to any of claims 18 to 30, further comprising

    - inputting information about a traveling deviation of an intrinsic physical quantity administered to the blood stream of said patient at a first location of the blood circulation of said patient, and
    - reading in measurement readings of said physical quantity at a second location of the blood circulation of said patient over the course of time,

    wherein, said measurement readings of said physical quantity at said second location of the blood circulation of said patient are recorded as a dilution curve, and said comparative value of at least one of said physiological parameters is determined from said dilution curve using dilution algorithms.

32. Method according to claim 30 or claim 31, wherein said comparative value is used for calibration.

33. Method according to claim 32, wherein calibration includes determining, using said comparative value, correction parameter $\alpha$ used in determining said model parameter, said correction parameter $\alpha$ assuming values greater than or equal to 1, said values being the higher the less the patient's heart frequency deviates from a rhythmic condition.

34. Method according to any of claims 30 to 33, wherein said physiological parameter is rejected and re-calculated, if the difference between the determined physiological parameter and the respective comparative value exceeds a threshold value.

35. Physical storage medium having stored thereon a computer program which, when executed on a computer system, causes the computer system to perform a method according to one of claims 18 to 34.


**Patentansprüche**

1. Vorrichtung zum Bestimmen mindestens eines physiologischen Parameters eines Patienten, wobei die Vorrichtung aufweist:

    - eine Drucksensorvorrichtung, die dazu ausgelegt ist, Messwerte eines arteriellen Blutdrucks des Patienten zu liefern,

- Speichermittel zum Speichern der Messwerte als eine Druckkurve P(t) über die Zeit t,
- Auswertungsmittel, die dazu ausgelegt sind, einen mittleren arteriellen Blutdruck <P> aus der Druckkurve P(t) zu bestimmen und den mindestens einen physiologischen Parameter unter der Verwendung des mittleren arteriellen Blutdrucks <P> zu bestimmen,

**dadurch gekennzeichnet, dass**

die Auswertungsmittel ferner dazu ausgelegt sind, - mindestens entweder eine spektrale Dichte $S(\omega)$ der Druckkurve oder eine Varianz $<(\delta P)^2>$ des arteriellen Blutdrucks zu bestimmen,

- mindestens einen Modellparameter, der einen Effektivwert eines Herzschlags repräsentiert, unter der Verwendung des mittleren arteriellen Blutdrucks <P> und mindestens entweder der spektralen Dichte $S(\omega)$ oder der Varianz $<(\delta P)^2>$ zu bestimmen, wobei der Effektivwert aus einer effektiven Amplitude $A_{eff}$ des Herzschlags, einer effektiven Dauer $d_{eff}$ des Herzschlags und einer effektiven Fläche $F_{eff}$ unter dem Herzschlag ausgewählt wird, und
- den mindestens einen physiologischen Parameter unter der Verwendung des mindestens einen Modellparameters zu bestimmen.

2. Vorrichtung gemäß Anspruch 1, wobei der mindestens eine physiologische Parameter mindestens entweder Schlagvolumen SV, Herzminutenvolumen HMV oder Ejektionsfraktion EF beinhaltet.

3. Vorrichtung gemäß Anspruch 2, wobei die Auswertungsmittel dazu ausgelegt sind, das Schlagvolumen SV als ein Produkt aus einem ersten Modellparameter, der die effektive Amplitude $A_{eff}$ repräsentiert, und einem zweiten Modellparameter, der die effektive Dauer $d_{eff}$ repräsentiert, zu bestimmen.

4. Vorrichtung gemäß Anspruch 2 oder Anspruch 3, wobei die Auswertungsmittel dazu ausgelegt sind, die Ejektionsfraktion EF als ein Produkt eines Modellparameters, der die effektive Dauer $d_{eff}$ repräsentiert, und einer Herzrate HR des Patienten zu bestimmen.

5. Vorrichtung gemäß einem der Ansprüche 2 bis 4, wobei die Auswertungsmittel dazu ausgelegt sind, das Herzminutenvolumen HMV als ein Produkt eines ersten Modellparameters, der die effektive Amplitude $A_{eff}$ repräsentiert, eines zweiten Modellparameters, der die effektive Dauer $d_{eff}$ repräsentiert, und einer Annäherung der Herzrate des Patienten zu bestimmen.

6. Vorrichtung gemäß Anspruch 5, wobei die Annäherung der Herzrate ausgewählt wird aus einer tatsächlich gemessenen Herzrate HR und einer Annäherungsfunktion $\lambda_{eff}$, wobei die Annäherungsfunktion $\lambda_{eff}$ einen Quotienten mit einem Dividenden, der das Quadrat des mittleren arteriellen Blutdrucks <P> aufweist, und einem Divisor enthält, der die spektrale Dichte $S(\omega)$, bei $\omega = 0$, aufweist.

7. Vorrichtung gemäß einem der vorhergehenden Ansprüche, wobei die Auswertungsmittel dazu ausgelegt sind, einen Korrekturparameter $\alpha$ beim Bestimmen des Modellparameters zu verwenden, wobei der Korrekturparameter $\alpha$ Werte annimmt, die größer oder gleich 1 sind, wobei die Wert umso größer sind, je weniger die Herzfrequenz des Patienten von einer rhythmischen Bedingung abweicht.

8. Vorrichtung gemäß Anspruch 7, wobei die Auswertungsmittel dazu ausgelegt sind, eine monotone Korrekturfunktion $\sigma$ zu verwenden, die von dem Korrekturparameter $\alpha$ abhängt und Werte von 0 bis 1 annimmt, wobei die Korrekturfunktion $\sigma$ den Wert 0 annimmt, wenn der Korrekturparameter $\alpha$ gleich 1 ist, und die Korrekturfunktion $\sigma$ den Wert 1 annimmt, wenn der Korrekturparameter $\alpha$ gegen unendlich strebt.

9. Vorrichtung gemäß Anspruch 8, wobei die effektive Amplitude $A_{eff}$ als ein Quotient mit einem Dividenden, der die Summe der Varianz $<(\delta P)^2>$ und des Produkts der Korrekturfunktion $\sigma$ und des Quadrats des mittleren arteriellen Drucks <P> aufweist, und einem Divisor, der den mittleren arteriellen Druck <P> aufweist, vorgesehen ist.

10. Vorrichtung gemäß Anspruch 8 oder Anspruch 9, wobei die effektive Amplitude $d_{eff}$ als ein Quotient mit einem Dividenden, der die spektrale Dichte $S(w)$, bei $w = 0$, aufweist, und einem Divisor, der die Summe der Varianz $<(\delta P)^2>$ und des Produkts der Korrekturfunktion $\sigma$ und des Quadrats des mittleren arteriellen Drucks <P> aufweist, vorgesehen ist.

11. Vorrichtung gemäß einem der vorhergehenden Ansprüche, aufweisend Fourier-Transformationsmittel zum Bestimmen der spektralen Dichte S(w) als die Fourier-Transformation der Autokorrelation der Druckkurve.

12. Vorrichtung gemäß einem der vorhergehenden Ansprüche, wobei die spektrale Dichte S(w), bei w = 0, als die Varianz $<(\delta P)^2>$, multipliziert mit einem konstanten Faktor, vorgesehen ist.

13. Vorrichtung gemäß einem der vorhergehenden Ansprüche, wobei die Auswertungseinheit ferner dazu ausgelegt ist, einen Vergleichswert mindestens eines der physiologischen Parameter aus der Druckkurve unter der Verwendung von Pulskonturalgorithmen zu bestimmen.

14. Vorrichtung gemäß einem der vorhergehenden Ansprüche, ferner aufweisend:

- Mittel zum Verabreichen einer wandernden Abweichung einer intrinsischen physikalischen Größe an den Blutstrom des Patienten an einem ersten Ort der Blutzirkulation des Patienten, und
- Sensormittel zum Messen der physikalischen Größe an einem zweiten Ort der Blutzirkulation des Patienten über den Verlauf der Zeit,

wobei die Speichermittel dazu ausgelegt sind, die über den Verlauf der Zeit an dem zweiten Ort gemessene physikalische Größe als eine Dilutionskurve aufzuzeichnen, und die Auswertungseinheit dazu ausgelegt ist, unter der Verwendung von Dilutionsalgorithmen aus der Dilutionskurve einen Vergleichswert mindestens eines der physiologischen Parameter zu bestimmen.

15. Vorrichtung gemäß Anspruch 13 oder Anspruch 14, wobei die Auswertungseinheit dazu ausgelegt ist, den Vergleichswert zur Kalibrierung zu verwenden.

16. Vorrichtung gemäß Anspruch 15, wobei die Kalibrierung beinhaltet, dass unter der Verwendung des Vergleichswerts der Korrekturparameter $\alpha$ bestimmt wird, der beim Bestimmen des Modellparameters verwendet wird, wobei der Korrekturparameter $\alpha$ Werte annimmt, die größer oder gleich 1 sind, wobei die Werte umso größer sind, je weniger die Herzfrequenz des Patienten von einer rhythmischen Bedingung abweicht.

17. Vorrichtung gemäß einem der Ansprüche 13 bis 16, wobei die Auswertungseinheit dazu ausgelegt ist, den physiologischen Parameter zu verwerfen und neu zu berechnen, wenn die Differenz zwischen dem bestimmten physiologischen Parameter und dem jeweiligen Vergleichswert einen Schwellenwert übersteigt.

18. Verfahren zum Bestimmen mindestens eines physiologischen Parameters eines Patienten, wobei das Verfahren die folgenden Schritte aufweist:

- Importieren von Messwerten eines arteriellen Blutdrucks des Patienten,
- Speichern der Messwerte als eine Druckkurve P(t) über die Zeit t,
- Bestimmen eines mittleren arteriellen Blutdrucks <P> aus der Druckkurve P(t) und Bestimmen mindestens eines physiologischen Parameters unter der Verwendung des mittleren arteriellen Blutdrucks <P>,

**dadurch gekennzeichnet, dass**
das Verfahren ferner beinhaltet:

- Bestimmen mindestens entweder einer spektralen Dichte S(w) der Druckkurve und einer Varianz $<(\delta P)^2>$ des arteriellen Blutdrucks,
- Bestimmen mindestens eines Modellparameters, der einen Effektivwert eines Herzschlags repräsentiert, unter der Verwendung des mittleren arteriellen Blutdrucks <P> und mindestens entweder der spektralen Dichte S(w) oder der Varianz $<(\delta P)^2>$, wobei der Effektivwert aus einer effektiven Amplitude $A_{eff}$ des Herzschlags, einer effektiven Dauer $d_{eff}$ des Herzschlags und einer effektiven Fläche $F_{eff}$ unter dem Herzschlag ausgewählt wird, und
- Bestimmen des mindestens einen physiologischen Parameters unter der Verwendung des mindestens einen Modellparameters.

19. Verfahren gemäß Anspruch 18, wobei der mindestens eine physiologische Parameter mindestens entweder Schlagvolumen SV, Herzminutenvolumen HMV oder Ejektionsfraktion EF beinhaltet.

20. Verfahren gemäß Anspruch 19, wobei das Schlagvolumen SV als ein Produkt aus einem ersten Modellparameter, der die effektive Amplitude $A_{eff}$ repräsentiert, und einem zweiten Modellparameter, der die effektive Dauer $d_{eff}$ repräsentiert, bestimmt wird.

21. Verfahren gemäß Anspruch 19 oder Anspruch 20, wobei die Ejektionsfraktion EF als ein Produkt eines Modellparameters, der die effektive Dauer $d_{eff}$ repräsentiert, und einer Herzrate HR des Patienten bestimmt wird.

22. Verfahren gemäß einem der Ansprüche 19 bis 21, wobei das Herzminutenvolumen HMV als ein Produkt eines ersten Modellparameters, der die effektive Amplitude $A_{eff}$ repräsentiert, eines zweiten Modellparameters, der die effektive Dauer $d_{eff}$ repräsentiert, und einer Annäherung der Herzrate des Patienten bestimmt wird.

23. Verfahren gemäß Anspruch 22, wobei die Annäherung der Herzrate ausgewählt wird aus einer tatsächlich gemessenen Herzrate HR und einer Annäherungsfunktion $\lambda_{eff}$, wobei die Annäherungsfunktion $\lambda_{eff}$ einen Quotienten mit einem Dividenden, der das Quadrat des mittleren arteriellen Blutdrucks <P> aufweist, und einem Divisor enthält, der die spektrale Dichte S(w), bei w = 0, aufweist.

24. Verfahren gemäß einem der Ansprüche 18 bis 23, wobei ein Korrekturparameter $\alpha$ beim Bestimmen des Modellparameters verwendet wird, wobei der Korrekturparameter $\alpha$ Werte annimmt, die größer oder gleich 1 sind, wobei die Wert umso größer sind, je weniger die Herzfrequenz des Patienten von einer rhythmischen Bedingung abweicht.

25. Verfahren gemäß Anspruch 24, wobei eine monotone Korrekturfunktion $\sigma$ verwendet wird, die von dem Korrekturparameter $\alpha$ abhängt und Werte von 0 bis 1 annimmt, wobei die Korrekturfunktion $\sigma$ den Wert 0 annimmt, wenn der Korrekturparameter $\alpha$ gleich 1 ist, und die Korrekturfunktion $\sigma$ den Wert 1 annimmt, wenn der Korrekturparameter $\alpha$ gegen unendlich strebt.

26. Verfahren gemäß Anspruch 25, wobei die effektive Amplitude $A_{eff}$ als ein Quotient vorgesehen ist, wobei der Dividend des Quotienten die Summe der Varianz $<(\delta P)^2>$ und des Produkts der Korrekturfunktion $\sigma$ und des Quadrats des mittleren arteriellen Drucks <P> aufweist, und der Divisor des Quotienten den mittleren arteriellen Druck <P> aufweist.

27. Verfahren gemäß Anspruch 25 oder Anspruch 26, wobei die effektive Amplitude $d_{eff}$ als ein Quotient mit einem Dividenden, der die spektrale Dichte S(w), bei w = 0, aufweist, und einem Divisor, der die Summe der Varianz $<(\delta P)^2>$ und des Produkts der Korrekturfunktion $\sigma$ und des Quadrats des mittleren arteriellen Drucks <P> aufweist, vorgesehen ist.

28. Verfahren gemäß einem der Ansprüche 18 bis 27, wobei die spektrale Dichte S(w) als die Fourier-Transformation der Autokorrelation der Druckkurve bestimmt wird.

29. Verfahren gemäß einem der Ansprüche 18 bis 28, wobei die spektrale Dichte S(w), bei w = 0, als die Varianz $<(\delta P)^2>$, multipliziert mit einem konstanten Faktor, vorgesehen ist.

30. Verfahren gemäß einem der Ansprüche 18 bis 29, wobei ein Vergleichswert mindestens eines der physiologischen Parameter aus der Druckkurve unter der Verwendung von Pulskonturalgorithmen bestimmt wird.

31. Verfahren gemäß einem der Ansprüche 18 bis 30, ferner aufweisend:

- Eingeben von Informationen über eine wandernde Abweichung einer intrinsischen physikalischen Größe, die an einen Blutstrom des Patienten an einem ersten Ort der Blutzirkulation des Patienten verabreicht wird, und
- Einlesen von Messwerten der physikalischen Größe an einem zweiten Ort der Blutzirkulation des Patienten über den Verlauf der Zeit,

wobei die Messwerte der physikalischen Größe an dem zweiten Ort der Blutzirkulation des Patienten als eine Dilutionskurve aufgezeichnet werden, und der Vergleichswert mindestens eines der physiologischen Parameter unter der Verwendung von Dilutionsalgorithmen aus der Dilutionskurve bestimmt wird.

32. Verfahren gemäß Anspruch 30 oder Anspruch 31, wobei der Vergleichswert zur Kalibrierung verwendet wird.

33. Verfahren gemäß Anspruch 32, wobei die Kalibrierung beinhaltet, dass unter der Verwendung des Vergleichswerts der Korrekturparameter $\alpha$ bestimmt wird, der beim Bestimmen des Modellparameters verwendet wird, wobei der

Korrekturparameter $\alpha$ Werte annimmt, die größer oder gleich 1 sind, wobei die Werte umso größer sind, je weniger die Herzfrequenz des Patienten von einer rhythmischen Bedingung abweicht.

34. Verfahren gemäß einem der Ansprüche 30 bis 33, wobei der physiologische Parameter verworfen und neu berechnet wird, wenn die Differenz zwischen dem bestimmten physiologischen Parameter und dem jeweiligen Vergleichswert einen Schwellenwert übersteigt.

35. Physisches Speichermedium, auf dem ein Computerprogramm gespeichert ist, das bei seiner Ausführung auf einem Computersystem das
Computersystem dazu veranlasst, ein Verfahren gemäß einem der Ansprüche 18 bis 34 durchzuführen.

**Revendications**

1. Appareil pour la détermination d'au moins un paramètre physiologique d'un patient, ledit appareil comprenant :

   - un dispositif capteur de pression conçu pour fournir des lectures d'une pression artérielle dudit patient,
   - des moyens de mémoire pour stocker lesdites lectures sous la forme d'une courbe de pression P(t) en fonction du temps t,
   - des moyens d'évaluation conçus pour déterminer une pression artérielle moyenne <P> à partir de ladite courbe de pression P(t) et pour déterminer ledit ou lesdits paramètres physiologiques à l'aide de ladite pression artérielle moyenne <P>,

   **caractérisé par le fait que** :

   lesdits moyens d'évaluation sont en outre conçus pour :

   - déterminer au moins l'une d'une densité spectrale S ($\omega$) de ladite courbe de pression et d'une variance <($\delta P)^2$> de ladite pression artérielle,
   - déterminer au moins un paramètre de modèle représentant une valeur efficace d'un battement cardiaque à l'aide de ladite pression artérielle moyenne <P> et d'au moins l'une de ladite tension spectrale S($\omega$) et de ladite variance <($\delta P)^2$>, ladite valeur efficace étant choisie parmi une amplitude efficace $A_{eff}$ dudit battement cardiaque, une durée efficace $d_{eff}$ dudit battement cardiaque et une aire efficace $F_{eff}$ sous ledit battement cardiaque, et
   - déterminer ledit ou lesdits paramètres physiologiques à l'aide dudit ou desdits paramètres de modèle.

2. Appareil selon la revendication 1, dans lequel ledit ou lesdits paramètres physiologiques comprennent au moins l'un du volume d'éjection systolique SV, du débit cardiaque CO et de la fraction d'éjection EF.

3. Appareil selon la revendication 2, dans lequel lesdits moyens d'évaluation sont conçus pour déterminer ledit volume d'éjection systolique SV comme étant un produit d'un premier paramètre de modèle représentant ladite amplitude efficace $A_{eff}$ et d'un second paramètre de modèle représentant ladite durée efficace $d_{eff}$.

4. Appareil selon la revendication 2 ou la revendication 3, dans lequel lesdits moyens d'évaluation sont conçus pour déterminer ladite fraction d'éjection EF comme étant un produit d'un paramètre de modèle représentant ladite durée efficace $d_{eff}$ et d'une fréquence cardiaque HR dudit patient.

5. Appareil selon l'une des revendications 2 à 4, dans lequel lesdits moyens d'évaluation sont conçus pour déterminer ledit débit cardiaque CO comme étant un produit d'un premier paramètre de modèle représentant ladite amplitude efficace $A_{eff}$, d'un second paramètre de modèle représentant ladite durée efficace $d_{eff}$ et d'une approximation d'une fréquence cardiaque dudit patient.

6. Appareil selon la revendication 5, dans lequel ladite approximation de ladite fréquence cardiaque est choisie parmi une fréquence cardiaque mesurée réelle HR et une fonction d'approximation $\lambda_{eff}$, ladite fonction d'approximation $\lambda_{eff}$ comprenant un quotient avec un dividende comprenant le carré de la pression artérielle moyenne <P> et un diviseur comprenant la densité spectrale S ($\omega$) à $\omega = 0$.

7. Appareil selon l'une quelconque des revendications précédentes, dans lequel lesdits moyens d'évaluation sont conçus pour utiliser un paramètre de correction $\alpha$ dans la détermination dudit paramètre de modèle, ledit paramètre de correction $\alpha$ prenant des valeurs supérieures ou égales à 1, plus lesdites valeurs étant élevées, moins la fréquence cardiaque du patient s'écartant d'un état rythmique.

**8.** Appareil selon la revendication 7, dans lequel lesdits moyens d'évaluation sont conçus pour utiliser une fonction de correction monotone $\sigma$ dépendant dudit paramètre de correction $\alpha$ et prenant des valeurs allant de 0 à 1, ladite fonction de correction $\sigma$ prenant la valeur 0 si ledit paramètre de correction $\alpha$ est égal à 1 et ladite fonction de correction $\sigma$ prenant la valeur 1 pour ledit paramètre de correction $\alpha$ tendant vers l'infini.

**9.** Appareil selon la revendication 8, dans lequel ladite amplitude efficace $A_{eff}$ est fournie sous la forme d'un quotient avec un dividende comprenant la somme de ladite variance $\langle(\delta P)^2\rangle$ et le produit de ladite fonction de correction $\sigma$ et du carré de la pression artérielle moyenne $\langle P\rangle$ et un diviseur comprenant la pression artérielle moyenne $\langle P\rangle$.

**10.** Appareil selon la revendication 8 ou la revendication 9, dans lequel ladite amplitude efficace $A_{eff}$ est fournie sous la forme d'un quotient avec un dividende comprenant la densité spectrale $S(\omega)$ à $\omega = 0$ et un diviseur comprenant la somme de ladite variance $\langle(\delta P)^2\rangle$ et le produit de ladite fonction de correction $\sigma$ et du carré de la pression artérielle moyenne $\langle P\rangle$.

**11.** Appareil selon l'une quelconque des revendications précédentes, comprenant des moyens de Transformation de Fourier pour déterminer ladite densité spectrale $S(\omega)$ comme étant la Transformée de Fourier de l'autocorrélation de ladite courbe de tension.

**12.** Appareil selon l'une quelconque des revendications précédentes, dans lequel la densité spectrale $S(\omega)$ à $\omega = 0$ est fournie comme étant ladite variance $\langle(\delta P)^2\rangle$ multipliée par un facteur constant.

**13.** Appareil selon l'une quelconque des revendications précédentes, dans lequel ladite unité d'évaluation est en outre conçue pour déterminer une valeur comparative d'au moins l'un desdits paramètres physiologiques à partir de ladite courbe de pression à l'aide d'algorithmes de contour de pouls.

**14.** Appareil selon l'une quelconque des revendications précédentes, comprenant en outre :

- des moyens pour administrer un écart de déplacement d'une quantité physique intrinsèque à la circulation sanguine dudit patient en un premier emplacement de la circulation sanguine dudit patient, et
- des moyens capteurs pour mesurer au cours du temps ladite quantité physique en un second emplacement de la circulation sanguine dudit patient,

lesdits moyens de mémoire étant conçus pour enregistrer ladite quantité physique mesurée au cours du temps audit second emplacement sous la forme d'une courbe de dilution, et ladite unité d'évaluation étant conçue pour déterminer une valeur comparative d'au moins l'un desdits paramètres physiologiques à partir de ladite courbe de dilution à l'aide d'algorithmes de dilution.

**15.** Appareil selon la revendication 13 ou la revendication 14, dans lequel ladite unité d'évaluation est conçue pour utiliser ladite valeur comparative pour l'étalonnage.

**16.** Appareil selon la revendication 15, dans lequel l'étalonnage comprend la détermination, à l'aide de ladite valeur comparative, d'un paramètre de correction $\alpha$ utilisé dans la détermination dudit paramètre de modèle, ledit paramètre de correction $\alpha$ prenant des valeurs supérieures ou égales à 1, plus lesdites valeurs étant élevées, moins la fréquence cardiaque du patient s'écartant d'un état rythmique.

**17.** Appareil selon l'une quelconque des revendications 13 à 16, dans lequel ladite unité d'évaluation est conçue pour rejeter et recalculer ledit paramètre physiologique, si la différence entre le paramètre physiologique déterminé et la valeur comparative respective dépasse une valeur de seuil.

**18.** Procédé de détermination d'au moins un paramètre physiologique d'un patient, ledit procédé comprenant les étapes consistant à :

- importer des lectures d'une pression artérielle dudit patient,
- stocker lesdites lectures sous forme d'une courbe de pression $P(t)$ en fonction du temps $t$,

- déterminer une pression artérielle moyenne <P> à partir de ladite courbe de pression P(t) et déterminer au moins un paramètre physiologique à l'aide de ladite pression artérielle moyenne <P>

**caractérisé par le fait que**

ledit procédé comprend en outre :

- la détermination d'au moins l'une d'une densité spectrale S ($\omega$) de ladite courbe de pression et d'une variance <($\delta$P)$^2$> de ladite pression artérielle,
- la détermination d'au moins un paramètre de modèle représentant une valeur efficace d'un battement cardiaque à l'aide de ladite pression artérielle moyenne <P> et d'au moins l'une de ladite densité spectrale S ($\omega$) et de ladite variance <($\delta$P)$^2$>, ladite valeur efficace étant choisie parmi une amplitude efficace $A_{eff}$ dudit battement cardiaque, une durée efficace $d_{eff}$ dudit battement cardiaque et une aire efficace $F_{eff}$ sous ledit battement cardiaque, et
- la détermination dudit ou desdits paramètres physiologiques à l'aide dudit ou desdits paramètres de modèle.

**19.** Procédé selon la revendication 18, dans lequel ledit ou lesdits paramètres physiologiques comprennent au moins l'un du volume d'éjection systolique SV, du débit cardiaque CO et de la fraction d'éjection EF.

**20.** Procédé selon la revendication 19, dans lequel ledit volume d'éjection systolique SV est déterminé comme étant un produit d'un premier paramètre de modèle représentant ladite amplitude efficace $A_{eff}$ et d'un second paramètre de modèle représentant ladite durée efficace $d_{eff}$.

**21.** Procédé selon la revendication 19 ou la revendication 20, dans lequel ladite fraction d'éjection EF est déterminée comme étant un produit d'un paramètre de modèle représentant ladite durée efficace $d_{eff}$ et d'une fréquence cardiaque HR dudit patient.

**22.** Procédé selon l'une des revendications 19 à 21, dans lequel ledit débit cardiaque CO est déterminé comme étant un produit d'un premier paramètre de modèle représentant ladite amplitude efficace $A_{eff}$, d'un second paramètre de modèle représentant ladite durée efficace $d_{eff}$ et d'une approximation d'une fréquence cardiaque dudit patient.

**23.** Procédé selon la revendication 22, dans lequel ladite approximation de ladite fréquence cardiaque est choisie parmi une fréquence cardiaque mesurée réelle HR et une fonction d'approximation $\lambda_{eff}$, ladite fonction d'approximation $\lambda_{eff}$ comprenant un quotient avec un dividende comprenant le carré de la pression artérielle moyenne <P> et un diviseur comprenant la densité spectrale S ($\omega$) à $\omega$ = 0.

**24.** Procédé selon l'une quelconque des revendications 18 à 23, dans lequel un paramètre de correction $\alpha$ est utilisé dans la détermination dudit paramètre de modèle, ledit paramètre de correction $\alpha$ prenant des valeurs supérieures ou égales à 1, plus lesdites valeurs étant élevées, moins la fréquence cardiaque du patient s'écartant d'un état rythmique.

**25.** Procédé selon la revendication 24, dans lequel une fonction de correction monotone $\sigma$ est utilisée en fonction dudit paramètre de correction $\alpha$ et prend des valeurs allant de 0 à 1, ladite fonction de correction $\sigma$ prenant la valeur 0 si ledit paramètre de correction $\alpha$ est égal à 1 et ladite fonction de correction $\sigma$ prenant la valeur 1 pour ledit paramètre de correction $\alpha$ tendant vers l'infini.

**26.** Procédé selon la revendication 25, dans lequel ladite amplitude efficace $A_{eff}$ est fournie sous la forme qu'un quotient, le dividende dudit quotient comprenant la somme de ladite variance <($\delta$P)$^2$> et du produit de ladite fonction de correction $\sigma$ et du carré de la pression artérielle moyenne <P> et le diviseur dudit quotient comprenant la pression artérielle moyenne <P>.

**27.** Procédé selon la revendication 25 ou la revendication 26, dans lequel ladite amplitude efficace $A_{eff}$ est fournie sous la forme d'un quotient avec un dividende comprenant la densité spectrale S ($\omega$) à $\omega$ = 0 et un diviseur comprenant la somme de ladite variance <($\delta$P)$^2$> et du produit de ladite fonction de correction $\sigma$ et du carré de la pression artérielle moyenne <P>.

**28.** Procédé selon l'une quelconque des revendications 18 à 27, dans lequel ladite densité spectrale S ($\omega$) est déterminée comme étant la Transformée de Fourier de l'autocorrélation de ladite courbe de pression.

**29.** Procédé selon l'une quelconque des revendications 18 à 28, dans lequel la densité spectrale S ($\omega$) à $\omega = 0$ est fournie comme étant ladite variance $<(\delta P)^2>$ multipliée par un facteur constant.

**30.** Procédé selon l'une quelconque des revendications 18 à 29, dans lequel une valeur comparative d'au moins l'un desdits paramètres physiologiques est déterminée à partir de ladite courbe de pression à l'aide d'algorithmes de contour de pouls.

**31.** Procédé selon l'une quelconque des revendications 18 à 30, comprenant en outre :

- l'entrée d'informations concernant un écart de déplacement d'une quantité physique intrinsèque administrée à la circulation sanguine dudit patient à un premier emplacement de la circulation sanguine dudit patient, et
- la lecture dans des lectures de mesure de ladite quantité physique en un second emplacement de la circulation sanguine dudit patient au cours du temps, lesdites lectures de mesure de ladite quantité physique audit second emplacement de la circulation sanguine dudit patient étant enregistrées sous la forme d'une courbe de dilution, et ladite valeur comparative d'au moins l'un desdits paramètres physiologiques étant déterminée à partir de ladite courbe de dilution à l'aide d'algorithmes de dilution.

**32.** Procédé selon la revendication 30 ou la revendication 31, dans lequel ladite valeur comparative est utilisée pour l'étalonnage.

**33.** Procédé selon la revendication 32, dans lequel l'étalonnage comprend la détermination, à l'aide de ladite valeur comparative, d'un paramètre de correction $\alpha$ utilisé dans la détermination dudit paramètre de modèle, ledit paramètre de correction $\alpha$ prenant des valeurs supérieures ou égales à 1, plus lesdites valeurs étant élevées, moins la fréquence cardiaque du patient s'écartant d'un état rythmique.

**34.** Procédé selon l'une quelconque des revendications 30 à 33, dans lequel ledit paramètre physiologique est rejeté et recalculé, si la différence entre le paramètre physiologique déterminé et la valeur comparative respective dépasse une valeur de seuil.

**35.** Support de stockage physique ayant, stocké sur celui-ci, un programme d'ordinateur qui, lorsqu'il est exécuté sur un système d'ordinateur, amène le système d'ordinateur à mettre en oeuvre un procédé selon l'une des revendications 18 à 34.

```
┌─────────────────────────┐
│ Measure arterial or aortic │
│ pressure P              │
└─────────────────────────┘
            │
            ▼
┌─────────────────────────┐
│ Calculate mean arterial  │
│ pressure <P>,           │─────────────┐
│ variance <(δP)²> and spectral │         │
│ density S(ω)            │              │
└─────────────────────────┘              ▼
                              ┌ ─ ─ ─ ─ ─ ─ ─ ─ ─ ─ ─ ─ ─ ┐
                                Determine HR from pressure P
                              └ ─ ─ ─ ─ ─ ─ ─ ─ ─ ─ ─ ─ ─ ┘
            │                            │
            ▼                            │
┌─────────────────────────┐             │
│ Select appropriate α, e.g. │◄──────────┘
│ α=1 or α = HR S(0)/[2<P>²] │────────────┐
└─────────────────────────┘               │
                                          ▼
                              ┌ ─ ─ ─ ─ ─ ─ ─ ─ ─ ─ ─ ─ ─ ┐
                                Derive effective heart rate λ_eff,
                                e.g. by 2 α <P>²/S(0)
                              └ ─ ─ ─ ─ ─ ─ ─ ─ ─ ─ ─ ─ ─ ┘
            │                            │
            ▼                            │
┌─────────────────────────┐             │
│ Derive CO from effective │◄────────────┘
│ area, e.g.              │
│ CO=HR·S(0)/(2α<P>) or    │
│ CO=λ_eff·S(0)/(2α<P>)    │
└─────────────────────────┘
```

Figure 1

Figure 2

Figure 3

Figure 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0920278 A
- EP 0947941 A

- EP 0637932 A

**Non-patent literature cited in the description**

- **N. R. Campbell.** The study of discontinuous phenomena. *Proc. Camp. Philos. Soc. Math. Phys. Sci.,* 1909, vol. 15, 117-136